Europäisches Patentamt

(19) European Patent Office

Office européen des brevets

(11) Publication number : **0 479 669 B1**

# (12) EUROPEAN PATENT SPECIFICATION

(45) Date of publication of patent specification :
**08.06.94 Bulletin 94/23**

(51) Int. Cl.⁵ : **A61K 7/043**

(21) Application number : **91402617.4**

(22) Date of filing : **01.10.91**

(54) **Nail enamel containing oxidized polyethylene coated inorganic pigments.**

(30) Priority : **01.10.90 US 591297**

(43) Date of publication of application :
**08.04.92 Bulletin 92/15**

(45) Publication of the grant of the patent :
**08.06.94 Bulletin 94/23**

(84) Designated Contracting States :
**AT BE CH DE DK ES FR GB GR IT LI NL SE**

(56) References cited :
**DE-A- 1 961 709
US-A- 3 864 294
US-A- 4 832 944**

(73) Proprietor : **L'OREAL
14, Rue Royale
F-75008 Paris (FR)**

(72) Inventor : **Weber, Robert A.
27-B S. Airmont Road
Suffern, New York, 10901 (US)**
Inventor : **Frankfurt, Christopher C.
1 Huxley Drive
Old Bridge, New Jersey, 08857 (US)**
Inventor : **Penicnak, John A.
151 Lookout Road
Mountain Lakes, New Jersey, 07046 (US)**

(74) Representative : **Stalla-Bourdillon, Bernard
CABINET NONY & CIE
29, rue Cambacérès
F-75008 Paris (FR)**

## Description

The present invention relates to a nail eneamel or lacquer. The invention particularly relates to nail enamels which are not only substantially free of settling and migration of the pigment and other materials suspended in a composition but are also substantially reduced or free from the flotation problem often found with nail enamels.

U.S. Patent N° 4,832,944 addresses the problem of preventing the settling and migration of pigments and other materials suspended in nail enamels. According to the patent, this is accomplished by utilizing inorganic pigments having a coating thereon consisting of organically substituted polysiloxanes which are chemically bonded to the pigment surface. Applicants have observed that the use of polysiloxane coated inorganic pigments does in fact substantially reduce the problems of pigment migration and settling. However, use of the polysiloxane coated pigments does not provide protection against the undersirable phenomenon known as flotation, a problem which is characterized by the appearance of a layer of clear liquid on the top of a bottle of nail enamel after a period of storage. The clear material does not contain pigments and thus must be reincorporated into the rest of the enamel by stirring or shaking before the enamel is used. This, of course, is undesirable.

BRIEF SUMMARY OF INVENTION

It has now been found that a nail enamel of greatly improved settling and migration characteristics and which is substantially free from the flotation problem is provided by formulating a nail enamel which contains an inorganic pigment that has been coated with oxidized polyethylene.

DETAILED DESCRIPTION OF THE INVENTION

The inorganic pigments which are useful in accordance with this invention are conventional nail enamel pigments which have been coated with oxidized polyethylene. One suitable source of such coated materials is the U.S. Cosmetics Corporation of Dayville, Connecticut. While the pigment can be supplied with levels of polyethylene treatment ranging from 1% to 10% by weight, a level in the range of from 1% to 4%, preferably 3% applied to the surface of the pigment has been found to provide good results.

Examples of such pigments include iron oxides, titanium dioxide, titanated mica, iron oxide, coated mica, ultramarine, chromium oxide, chromium hydroxide, manganese violet, and any other suitable recognized prior art pigment. Preferred among these are the iron oxide and titanium dioxide pigments.

In addition to the coated pigment, the nail enamel contains an otherwise conventional base including nitrocellulose, solvent, modifying resin, plasticizer, and suspending agent, and the nail enamels are prepared by conventional procedures. Examples of suitable enamel bases and procedures for preparing them are found in Cosmetics, Science and Technology, Edited by Balsam and Sagarin. 2nd Edition, Vol 2, Chapter 29 (Wiley Interscience, New York, New York).

The coated pigment is dispersed into the other nail enamel components by conventional techniques employed in nail enamel manufacture such as roller milling and chipping.

The table given in the Examples illustrates formulations that may be used and the differences between polyethylene treated, polysiloxane treated, and untreated inorganic pigments. Where the pigment is indicated as polyethylene treated, it has been coated with oxidized polyethylene and is available from U.S. Cosmetics, Connecticut as product number PI-T-3328 for the titanium dioxide and PT-C33-8075 and PT-B-335198 for the iron oxides. The pigment labelled polysiloxane treated is coated with poly(methylhydrogen) siloxane and was obtained from Clark Colors as product number 9454 for the coated iron oxides and product number 9428 for the coated titanium dioxide.

The numerical flotation evaluation ratings given in the table has the following meaning for stability :

1. Excellent      - No visible sign of flotation.
2. Good      - Very slight flotation.
3. Average      - Some flotation.
4. Poor      - Flotation readily apparent.
5. Unacceptable      - Very bad flotation.

The numerical evaluation ratings for migration and settling have the following meanings :

1. Excellent      - No migration or settling.
2. Good      - Very slight settling ; no migration.
3. Average      - Some settling ; very slight migration.
4. Poor      - Settling and migration readily apparent

2

5. Unacceptable         - Product not saleable.

<u>EXAMPLE</u>

Three samples of a nail enamel ("Whimsy Pink") were propared by uniformly mixing the ingredients given in the table below. The amounts are by weight. The products were compared for settling, migration, and flotation after 4 months storage without agitation at room temperature and at 45°C. Both polyethylene and polysiloxane are present on the treated pigment at a level of 3% by weight.

| Sample N° | | 1 | 2 | 3 |
|---|---|---|---|---|
| n-Butyl Acetate | | 25.00 | 25.00 | 25.00 |
| Toluene | | 25.00 | 25.00 | 25.00 |
| Ethyl Acetate | | 10.00 | 10.00 | 10.00 |
| Isopropyl Alcohol | | 6.75 | 6.75 | 6.75 |
| Bentone 27 (montmorillonite) | | 1.00 | 1.00 | 1.00 |
| Nitrocellulose | | 16.00 | 16.00 | 16.00 |
| Toluenesulfonamide formaldehyde resin | | 9.00 | 9.00 | 9.00 |
| Dibutyl phtalate | | 5.00 | 5.00 | 5.00 |
| Camphor | | 1.00 | 1.00 | 1.00 |
| Titanium dioxide | (polyethylene) | 1.00 | – | – |
| " | (polysiloxane) | – | 1.00 | – |
| " | (untreated) | – | – | 1.00 |
| Iron oxide, black | (polyethylene) | 0.10 | – | – |
| " | (polysiloxane) | – | 0.10 | – |
| " | (untreated) | – | – | 0.10 |
| Iron oxide, russet | (polyethylene) | 0.05 | – | – |
| " | (polysiloxane) | – | 0.05 | – |
| " | (untreated) | – | – | 0.05 |
| D & C Red N° 6, barium lake (untreated) | | 0.10 | 0.10 | 0.10 |

<u>Flotation observation after 4 mos.</u>

| | 1 | 2 | 3 |
|---|---|---|---|
| Room Temperature | 1 | 2 | 1 |
| 45°C | 2 | 3 | 2 |

<u>Settling, migration after 4 mos.</u>

| | 1 | 2 | 3 |
|---|---|---|---|
| Room Temperature | 1 | 1 | 3 |
| 45°C | 1 | 1 | 4 |

As can be seen from the results of this work, polysiloxane treatment (Sample N° 2) provides a good product which has no settling or migration of enamel ingredients on a 4-month storage period, both at room temperature and at 45°C. However, these products did not perform well in the flotation test, being evaluated at a 2 level at room temperature and at a 3 level at 45°C. The product of the present invention (Sample N° 1), in contrast, performed very well in the flotation test, as well as in the settling and migration tests.

Definitions

For the purpose of the present invention, the following terms are defined :

Settling - describes the problem noted in nail enamels when solids, including pigments, accumulate on the bottom of a bottle of nail enamel.

Migration - pigments in nail enamel form striations, marbleization and/or layering effects, leading to non-uniform appearance.

Flotation - this term describes the phenomenon where a clear liquid collects at the top of the nail enamel bottle.

## Claims

1. A nail enamel comprising at least one film forming material, at least one plasticizer, at least one solvent for the film forming material and plasticizer and at least one inorganic pigment, the inorganic pigment being one which has been treated with oxidized polyethylene to form a coating on the pigment particles.

2. The nail enamel of Claim 1, wherein the at least one inorganic pigment is treated to have up to 10% by weight of oxidized polyethylene coated on the surface of the pigment particles.

3. The nail enamel of Claim 1, wherein the inorganic pigment is treated to have from 1-10% by weight of oxidized polyethylene coated on the surface of the pigment particles.

4. The nail enamel of Claim 1, wherein the inorganic pigment is selected from the group consisting of iron oxides, titanated mica, titanium dioxide, ultramarine, chromium oxide, chromium hydroxide and manganese violet.

5. A method of preparing nail enamel containing at least one film-forming material, at least one plasticizer, at least one solvent for the film forming material and the plasticizer, and at least one inorganic pigment, the method comprising the step of dispersing an inorganic pigment having a surface treatment of oxidized polyethylene in a mixture of the film forming material, plasticizer and solvent.

6. The method of Claim 5, wherein the at least one inorganic pigment has up to 10% by weight of oxidized polyethylene.

7. The method of Claim 5, wherein the inorganic pigment is titanium dioxide and the pigment is treated to have 1-10% by weight of oxidized polyethylene coated or the surface of the pigment particles.

## Patentansprüche

1. Nagellack bestehend aus mindestens einem filmbildenden Material, mindestens einem Weichmacher, mindestens einem Lösungsmittel für das filmbildende Material und den Weichmacher sowie mindestens einem anorganischen Pigment, wobei das anorganische Pigment mit oxidiertem Polyethylen zur Bildung einer Beschichtung auf den Pigmentteilchen behandelt worden ist.

2. Nagellack gemäß Anspruch 1, bei dem das mindestens eine verwendete anorganische Pigment so behandelt wird, daß es mit bis zu 10 Gew.-% oxidiertem Polyethylen auf der Oberfläche der Pigmentteilchen beschichtet ist.

3. Nagellack gemäß Anspruch 1, bei dem das anorganische Pigment so behandelt wird, daß es mit 1 - 10 Gew.-% oxidiertem Polyethylen auf der Oberfläche der Pigmentteilchen beschichtet ist.

**4.** Nagellack gemäß Anspruch 1, bei dem das anorganische Pigment aus der aus Eisenoxiden, Titanglimmer, Titandioxid, Ultramarin, Chromoxid, Chromhydroxid und Manganviolett bestehenden Gruppe ausgewählt ist.

**5.** Verfahren zur Herstellung eines Nagellackes, der mindestens ein filmbildendes Material, mindestens einen Weichmacher, mindestens ein Lösungsmittel für das filmbildende Material und den Weichmacher sowie mindestens ein anorganisches Pigment enthält, wobei das Verfahren einen Dispergierschritt für das mit oxidiertem Polyethylen oberflächenbehandelte anorganische Pigment in einem Gemisch aus dem filmbildenden Material, dem Weichmacher und dem Lösungsmittel umfaßt.

**6.** Verfahren gemäß Anspruch 5, bei dem mindestens ein anorganisches Pigment bis zu 10 Gew.-% an oxidiertem Polyethylen aufweist.

**7.** Verfahren gemäß Anspruch 56, bei dem das anorganische Pigment Titandioxid ist und so behandelt wurde, daß es mit 1 - 10 Gew.-% oxidierten Polyethylens auf der Oberfläche der Pigmentteilchen beschichtet ist.

**Revendications**

**1.** Vernis à ongles comprenant au moins une substance filmogène, au moins un plastifiant, au moins un solvant pour la substance filmogène et le plastifiant, et au moins un pigment inorganique, le pigment inorganique étant traité avec un polyéthylène oxydé pour former un revêtement sur les particules de pigment.

**2.** Vernis à ongles selon la revendication 1, dans lequel ledit pigment inorganique est traité de façon à présenter jusqu'à 10 % en poids de polyéthylène oxydé formant un revêtement à la surface des particules de pigment.

**3.** Vernis à ongles selon la revendication 1, dans lequel ledit pigment inorganique est traité de façon à présenter de 1 à 10 % en poids de polyéthylène oxydé formant un revêtement à la surface des particules de pigment.

**4.** Vernis à ongles selon la revendication 1, dans lequel le pigment inorganique est choisi dans le groupe constitué par les oxydes de fer, le mica-titane, le dioxyde de titane, l'ultramarine, l'oxyde de chrome, l'hydroxyde de chrome et le violet de manganèse.

**5.** Procédé de préparation d'un vernis à ongles, comprenant au moins une substance filmogène, au moins un plastifiant, au moins un solvant pour la substance filmogène et le plastifiant, et au moins un pigment inorganique, ledit procédé consistant à disperser un pigment inorganique, ayant subi un traitement de surface avec un polyéthylène oxydé, dans un mélange de la substance filmogène, du plastifiant et du solvant.

**6.** Procédé selon la revendication 5, dans lequel ledit pigment inorganique a jusqu'à 10 % en poids de polyéthylène oxydé.

**7.** Procédé selon la revendication 5, dans lequel ledit pigment inorganique est du dioxyde de titane qui est traité de façon à avoir de 1 à 10 % en poids de polyéthylène oxydé revêtu sur la surface des particules de pigment.